# EUROPÄISCHE PATENTSCHRIFT

(11) **EP 0 205 513 B1**
(45) Veröffentlichungstag und Bekanntmachung des Hinweises auf die Patenterteilung: **17.03.1993**
(21) Anmeldenummer: 86900131.3
(22) Anmeldetag: 16.12.1985
(51) Int. Cl.: A61B 17/56, A61F 2/28

(54) **IMPLANTAT ZUR ARMIERUNG VON KNOCHEN UND ZUR VERANKERUNG VON KNOCHENSCHRAUBEN, IMPLANTATEN ODER IMPLANTATTEILEN IM KNOCHEN**
IMPLANT FOR ARMAMENT OF BONES AND FOR ANCHORING OF BONESCREWS, IMPLANTS OR PARTS OF IMPLANTS IN THE BONE
IMPLANT DESTINE A LA CONSOLIDATION OSSEUSE ET A L'ANCRAGE DANS L OS DE VIS, IMPLANTS ET PARTIES D'IMPLANT

(30) Priorität: 14.12.1984 DE 3445738
(43) Veröffentlichungstag der Anmeldung: 30.12.1986
(73) Patentinhaber: DRAENERT, Klaus, Dr.med., D-81545 München (DE)
(72) Erfinder: DRAENERT, Klaus, Dr.med., D-81545 München (DE)
(74) Vertreter: VOSSIUS & PARTNER
(86) Internationale Anmeldenummer: EP8500710
(87) Internationale Veröffentlichungsnummer: WO8603666

(56) Entgegenhaltungen:
- EP-A- 0 077 868
- DE-A- 1 965 350
- GB-A- 2 084 468
- US-A- 2 381 050
- Engineering in Medicine, vol 11, no 4, October 1982, D Raftopoulos et al: "A proposed design for an expanding hip nail"

## Beschreibung

Die Erfindung betrifft ein Implantat zur Armierung und/oder Verstärkung von Knochen und zur Verankerung von Knochenschrauben, Implantaten oder Implantatteilen im Knochen.

Bei Knochenfrakturen und Knochendefekten kann man verschiedene Heilungsformen erkennen. Nach dem histologischen Bild unterscheidet man die sogenannte spontane Knochenheilung von der primär knöchernen oder auch angiogenen Heilung. Die wesentlichen Unterschiede bestehen darin, daß bei der spontanen Knochenheilung Knorpel und Faserbindegewebe gebildet wird, das durch Umdifferenzierung in Geflechtknochen- und Lamellenknochengewebe umgewandelt wird, während bei der primär angiogenen Knochenheilung aufgrund der hohen mechanischen Stabilität bei großen Defekten primär Geflechtknochen bzw. bei kleinen Spalten direkt Lamellenknochen gebildet wird. Die Vorteile der primär angiogenen Knochenheilung liegen in der verkürzten Heilungszeit, d.h. in der früh erreichten knöchernen Überbrückung, die eine sehr frühe Mobilisation und Übungsstabilität garantiert.

Bei der konservativen Frakturbehandlung kommt es weitgehend zu spontaner Knochenheilung und deshalb auch oft zu Komplikationen, wie einer verzögerten oder ausgebliebenen Knochenheilung oder Pseudarthrosenbildung.

Primär knöcherne oder primär angiogene Knochenheilung kann in der Regel nicht ohne operative Versorgung und stabile Fixation mit Schrauben und Platten erreicht werden. Die die mechanische Stabilität garantierende interfragmentäre Kompression wird erreicht durch Vorspannung der Platte mit einem Plattenspanngerät oder durch die sogenannte dynamic compression plate und durch die interfragmentäre Schraube, insbesondere eine Schraube mit Zug- und Gleitloch oder aber auch durch sog. "fixateurs externes".
Diese Operationstechniken wurden im wesentlichen durch die Arbeitsgemeinschaft für Osteosynthesefragen erarbeitet. Das histologische Bild dieser Knochenheilung ist die callusfreie bzw. callusarme primär angiogene Knochenheilung mit Durchbauung der Havers'schen Systeme in Höhe des Frakturspaltes.

Die im Knochen verankerten Knochenschrauben finden in kompakten Rindenknochen mit vorgeschnittenem Gewinde einen festen Halt.In den metaphysären Knochenabschnitten, die von einem Schwammknochen ausgefüllt sind, finden sogenannte Spongiosaschrauben Anwendung, deren Gewinde weit ausladen und eine nahezu rechtwinklige Druckauflage aufweisen und sich in den Knochenbälkchen verhaken sollen.

Zugversuche und Drehmomentmessungen zeigen jedoch, daß der spongiöse Knochen der Metaphysen und der gelenknahen Abschnitte den Schrauben keinerlei Widerstand bietet, und die Schrauben in dem nachgebenden Spongiosaknochen keine Zugwirkung entfalten können. Die durch die Schrauben erzeugte Zugspannung wird innerhalb einiger Sekunden abgebaut; die Schrauben verhelfen zu keiner mechanischen Fixation der Fragmente, da sie keine interfragmentäre Kompression ausüben. Dies ist vor allem dort von entscheidender praktischer Bedeutung, wo es darauf ankommt, ungünstige Frakturspalten durch interfragmentären Schraubenzug aufeinander zu fixieren, wie dies z.B. bei bestimmten Formen von Schenkelhalsfrakturen notwendig ist. Die Folge ungenügender Fixation in diesem Bereich ist oftmals die Nekrose des gesamten Epiphysenabschnittes, nämlich die Schenkelhalskopfnekrose, die oft schon bei jungen Patienten einen prothetischen Ersatz erforderlich macht. Die sogenannten Spongiosaschrauben finden lediglich bei sehr jungen Individuen in dem spongiösen Knochen der Metaphysen Halt, so daß für die Dauer einiger Minuten eine echte Zugspannung nachgewiesen werden kann.

Bei älteren und alten Menschen kommt es sehr oft zu einer Rarefizierung der Knochenbälkchen, so daß normale Schrauben auch im Kompaktaknochen keinen Halt mehr finden. In der Praxis wurde in diesem Fall oft versucht, die Osteosynthese mit Platten und Schrauben durch einen Verbund mit Knochenzement zu verstärken, indem die Markhöhle mit Knochenzement ausgegossen und im aushärtenden Zement die Knochenschrauben fixiert wurden. Der Nachteil dieses Verfahrens liegt vor allem bei jüngeren Patienten darin, daß der Knochenzement nicht mehr entfernbar ist und als Fremdkörper für den Rest des Lebens im Markhöhlenabschnitt verweilt.

Ähnliche Probleme ergeben sich bei der Behandlung von Epiphysenverschlüssen. Bei einseitigen Epiphysenfrakturen, als deren Folge sehr oft ein ungleiches Wachstum in Kauf genommen werden muß, kann kontralateral die Epiphysenfuge ebenfalls verblockt werden. Die Schraubenfixation genügt zwar manchmal, um dieses Ergebnis zu erreichen, oft wird jedoch durch ungleichen Schraubenzug nur ein Teilverschluß der Epiphysenfuge erreicht, was ein Schiefwachstum zur Folge hat.

Ähnlich gelagerte Probleme treten ebenfalls auf bei der Durchführung von Arthrodesen, d.h. künstlich herbeigeführten Gelenkversteifungen in Fällen, in denen eine Gelenkbeweglichkeit zugunsten einer Gelenkstabilität nicht mehr wünschenswert ist. Solche Arthrodesen konnten bisher mit Schrauben nur selten erreicht werden, da die Schrauben mit der Zeit auslockerten.

Eine ähnlich gelagerte Problematik liegt darin, drohende Einbrüche tragender Epiphysenstrukturen oder drohende Schenkelhalskopfnekrosen, an die sich meist die vollständige Zerstörung des Hüftgelenkes anschließt, zu vermeiden; beispielsweise posttraumatisch eingedrückte Knochenpartien zu unterstützen oder anzuheben oder abgerissene knöcherne Sehnenansätze oder Bandinsertionen wieder zu reinserieren.

Aus der GB-A-2 084 468 ist ein chirurgisches Implantat in Form eines Bolzens bekannt, welcher in einem Loch in einem Knochen verankert werden soll. Der Bolzen weist einen rohrförmigen zylindrischen Körper und an einem Ende einen Kopf auf. Im Innern des Bolzens verläuft eine Durchführung, in die von dem den Kopf aufweisenden Ende her ein Stift eingeführt wird, durch welchen der Körper in Querrichtung ausgedehnt wird. Der Körper und der Stift bestehen aus einem resorbierbaren Material mit eingelagerten Kohlenstoffasern. Das zweiteilige Implantat gemäß GB-A-2 084 468 dient im wesentlichen als Anker im Rahmen eines Kits zum Fixieren einer Platte oder eines Bandes am Knochen. Aufgrund des Kopfes ist das Implantat nicht vollständig in den Knochen einführbar.

Aus der DE-B-19 65 350 ist ein Verbindungselement für Knochenfrakturen bekannt. Das Verbindungselement besteht aus einer in die Knochenbruchstücke einsetzbaren Dübelhülse mit einem mittels eines Spreizstiftes aufspreizbaren Spreizteil und einer auf das dem Spreizteil abgewandten Ende der Dübelhülse ansetzbaren Kopfschraube. Die Kopfschraube ist in die Dübelhülse einschraubbar, und der Spreizstift weist dasselbe Gewinde auf wie die Kopfschraube. Das Verbindungselement gemäß DE-B-19 65 350 besteht aus drei Teilen und ist aus rostfreiem Stahl hergestellt und somit nicht resorbierbar.

Der Erfindung liegt somit die Aufgabe zugrunde, ein chirurgisches Material bereitzustellen, das eine sichere Fixierung von Knochenschrauben und Platten, insbesondere in den gelenknahen Abschnitten des Knochens, gewährleistet und mit dem gefährdete oder eingedrückte Knochenpartien unterstützt werden können.

Diese Aufgabe wird durch die Merkmale der Patentansprüche 1 oder 2 gelöst.

Die Erfindung geht von dem Grundgedanken aus, ein Implantat bereitzustellen, das nach dem Einbringen in einen Knochen dessen Armierung, Unterstützung und/oder Verstärkung bewirkt und das sowohl im umgebenden Knochen einen festen Halt findet als auch seinerseits beispielsweise eine eingedrehte Schraube langzeitig zugfest aufnimmt. Der feste Halt im Knochen wird insbesondere dadurch gewährleistet, daß das Implantat sich entweder durch Eindrehen einer Schraube aufspreizt oder ausdehnt oder daß es aufgrund seiner Materialeigenschaften durch Kontakt mit Körperflüssigkeit im Knochen aufquillt; auf diese Weise wird ein Verhaken, Verblocken oder allgemein Festsitzen des als Implantat eingebrachten chirurgischen Materials gewährleistet. Das Implantat kann auch selbst im den Knochen eingedreht oder eingeschraubt werden, wenn aufgrund der Form und/oder des Materials des Implantats ein fester Halt im umgebenden Knochen gewährleistet ist.

Vorzugsweise ist die äußere Oberfläche des Implantates strukturiert, beispielsweise durch Schlitze und/oder Perforationen. Mit zunehmender Strukturierung wird die Verankerung des Implantats im Knochen verbessert und die Zugfestigkeit, wie die zum Herausziehen einer im Implantat verankerten Schraube erforderliche Kraft, erhöht.

Erfindungsgemäß ist das Implantat in Form eines Hohlkörpers ausgebildet. Durch Eindrehen einer Knochenschraube oder Verblocken eines Keils oder Zapfens oder eines Knochenzylinders, wie eines Knorpelknochenzylinders oder Bandknochenzylinders, oder durch Einstecken, Einschrauben oder Einspritzen eines austauschbaren Medikamententrägers und/oder Strahlentherapieträgers kann der Hohlkörper fest im Knochen verankert werden. Das Lumen des Hohlkörpers weist zu diesem Zweck eine geeignete Innenstruktur auf. Die Innenstruktur kann beispielsweise ein Innengewinde oder eine gewindeähnliche Struktur für eine Knochenschraube oder eine grobe, unregelmäßige Strukturierung mit Erhebungen und Vertiefungen sein, durch die ein eingeführter Keil oder Zylinder festgehalten und der Hohlkörper beim Einführen aufgedehnt wird. Besonders bevorzugt weist das Implantat die Form eines strukturierten, einseitig geschlossenen Hohlzylinders auf. Insbesondere ein derartiges Implantat wird nachstehend auch als Knochendübel bezeichnet.

Die erfindungsgemäßen, dübelartigen Implantate kommen überall da zur Anwendung, wo Osteotomien oder Frakturen den Bereich der Epiphyse und Metaphyse betreffen. Der Knochendübel wird durch ein Bohrloch in das jeweils kontralaterale Fragment plaziert und anschließend die Knochenschraube mit Unterlegscheibe eingedreht. Der Knochendübel sitzt danach sicher und fest in seiner Frakturhälfte und trägt so zum Aufbau der erforderlichen interfragmentären Kompression bei. Vorzugsweise ist der Innendurchmesser des Dübels etwas kleiner als der Außendurchmesser der einzudrehenden Spongiosaschraube, beispielsweise beträgt die Differenz etwa 2 mm. Auf diese Weise wird der Dübel beim Eindrehen der Schraube aufgespreizt und besonders stabil in der Spongiosa verblockt. Eine derart fixierte Spongiosaschraube weist eine beträchtlich erhöhte Langzeit-Zugfestigkeit auf, was sich anhand von Ausziehversuchen feststellen läßt.

Mit dem erfindungsgemäßen Knochendübel lassen sich auch bei ungünstigen Frakturspalten die einzelnen Fragmente mechanisch fixieren; dadurch wird beispielsweise bei Schenkelhalsfrakturen eine Nekrose des Epiphysenabschnittes verhindert. Mit dem erfindungsgemäßen Dübel-Implantat finden auch normale Spongiosaschrauben selbst in der rarefizierten Spongiosa des Schenkelhalskopfes bei älteren Menschen genügend Halt, und durch die stabile Verschraubung läßt sich die Fehlergebnisrate beträchtlich senken. Bei Verwendung des erfindungsgemäßen Dübel-Implantats genügen auch bei komplizierten Frakturtypen meist zwei oder drei Schrauben, um zu einer stabilen Schraubenfixation zu kommen.

Auch bei der Behandlung von Epiphysenverschlüssen und einseitigen Epiphysenfrakturen kann durch Verwendung des erfindungsgemäßen Knochendübels ein so hoher Druck in der Epiphysenfuge erzeugt werden, daß eine gleichmäßige Verknöcherung der Wachstumsfuge erfolgt.

Das erfindungsgemäße Dübel-Implantat ermöglicht weiterhin eine sichere Verblockung der Schrauben im rarefizierten, osteoporotischen Knochen bei älteren und alten Menschen, so daß auch in diesen Fällen auf eine Verbundosteosynthese unter Verwendung von Knochenzement zum Fixieren der Knochenschrauben verzichtet werden kann.

Mit dem erfindungsgemäßen Dübel-Implantat gelingt es auch, durch Verschrauben Arthrodesen durchzuführen, da durch die Verankerung der Schrauben im Implantat im Gelenkspalt derart hohe Drücke erzeugt werden, daß eine sofortige Verknöcherung einsetzt und die Durchbauung des Gelenkes mit knöchernen Strukturen erreicht werden kann.

Eine weitere Indikation der erfindungsgemäßen Dübel-Implantate besteht in der Unterstützung von vom Einbrechen bedrohten Segmenten, wie beispielsweise dem sphärischen Sektor des Schenkelhalskopfes bei der Kopfnekrose. Hierbei wird vorteilhafterweise eine Knochenschraube aus demselben Material wie das Implantat in den Dübel eingeschraubt, so daß der sphärische Sektor abgestützt wird. Auf diese Weise kann beispielsweise mit einem oder zwei Dübel-Implantaten die vollständige Ausheilung der drohenden Kopfnekrose erreicht werden. Die Kombination aus Schraube und Dübel-Implantat stützt die tragenden Epiphysenstrukturen wie ein Säulenfundament.

Eine besondere Applikationsform besteht für die mit einem Antibiotikum, wie Gentamycin bestückten Dübel-Implantate. Diese Implantate dienen nicht nur der stabilen Fixation und Verankerung von Knochenschrauben, sondern auch als sogenannte Drug-Applikatoren zur Bekämpfung des Osteitisinfektes.

Bei Bandausrissen und Reinsertionen von Sehnen und Apophysen sowie Epikondylen dient das erfindungsgemäße Dübel-Implantat direkt als Anker im spongiösen Knochen. Als besondere Indikationen treten hierzu beispielsweise noch Pfannenfixation bei Endoprothesen, Trochanterfixation bei Endoprothesen oder Trochanterfixation bei Reoperationen sowie allgemein die Verankerung von Komponenten künstlicher Gelenke, die eingeschraubt oder verblockt werden.

Eine besondere Dübelform stellt ein außen und innen strukturierter Hohlzylinder dar, der geeignet ist, Knochenzylinder, Knorpelknochenzylinder und Bandknochenzylinder stabil im Implantat zu fixieren. Sind diese Dübel perforiert und bestehen sie aus einem resorbierbaren Material, so führen sie zu einem schnellen knöchernen Einheilen des Transplantates oder reinserierten Knochenzylinders. Es können mit solchen Knochenzylindern in Verbindung mit den Dübeln Frakturspalte knöchern überbrückt werden.

Für die Applikation von Knochenschrauben im Spongiosabereich bei jüngeren Patienten Können die erfindungsgemäßen resorbierbaren Implantate verwendet werden Besorbierbare Materialien für Implantate sind als solche bekannt.

Das resorbierbare chirurgische Material zur Herstellung des erfindungsgemäßen Dübel-Implantates ist vorzugsweise auf der Basis von Polylactaten, Polyglykolaten, Polypeptiden, Kollagenen, Apatiten oder resorbierbaren Keramiken oder aus Verbunden von zwei oder mehr Materialien aufgebaut. Die vorstehenden resorbierbaren Materialien lassen sich teilweise leicht verflüssigen und im Gieß- oder Spritzgußverfahren verarbeiten, wobei alle gewünschten Formen herstellbar sind. Es lassen sich auf diese Weise sehr stabile, feste und widerstandsfähige Knochenimplantate herstellen, die ebenso einfach zu strukturieren sind wie mechanisch hergestellte Kunststoffdübel.

Vorzugsweise wird dem die resorbierbare Matrix des Implantates ausbildenden Basismaterial ein stabilisierender und bioaktivierender Füller zugesetzt, der vorzugsweise aus Hydroxylapatit oder resorbierbarem Trikalziumphosphat besteht, das gesintert oder geschlämmt ist. Der Füller wird vorzugsweise mit einem Anteil von 5 bis 50 Gewichtsprozent zugesetzt und führt zu einem beschleunigten Einbau des Implantats und zu einem verstärkten Knochenanbau. Der Füller kann beispielsweise zu der Schmelze des Basismaterials zugegeben und entweder ausgegossen oder in einer Spritzform unter Druck eingespritzt werden. Vorzugsweise wird der Füller als Kugeln mit einer Größe von etwa 10 bis 200 µm, vorzugsweise zwischen 15 und 30 µm, zugesetzt. Der Füller sollte ein hohes Porenvolumen aufweisen, wobei das Porenvolumen vorzugsweise etwa 25 bis 65 % seines Gesamtvolumens ausmacht, besonders bevorzugt mehr als 40%.

Die vorstehenden resorbierbaren Materialien weisen den Vorteil auf, daß sie im Kontakt mit der Körperflüssigkeit quellfähig sind, wobei die Kollagene noch stärkere Quellfähigkeit als die Polyglykolate aufweisen. Durch diese Quellfähigkeit wird einerseits das Dübel-Implantat hervorragend mechanisch im Knochen verankert, andererseits wird aufgrund der Quellung des Implantates das Gewebe einer Querdehnung ausgesetzt, was zu einem weiteren, starke knochenbildungsinduzierenden Effekt führt. Der durch die Dehnung des Implantats ausgeübte Reiz führt zu einem erheblich beschleunigten Knochenanbau, wie beispielsweise im Tierexperiment bestätigt wurde. Bereits nach wenigen Tagen werden direkte Knochenappositionen auf der Oberfläche des Implantates abgelagert und das Implantat vollständig entlang seiner gesamten Oberfläche stabil knöchern integriert. Eine in ein derartiges Dübel-Implantat eingedrehte Knochenschraube weist gegenüber nicht gedübelten Knochenschrauben im Auszugsversuch eine signifikant erhöhte Zugfestigkeit auf. Die vorstehenden resorbierbaren Materialien sind innerhalb von etwa 90 Tagen weitgehend resorbiert.

Um besonders stabile Implantate zu erzielen, kann das Implantatmaterial durch eine Armierung in Form von Fasern, Fasernetzen oder Geweben verstärkt werden, die ebenfalls aus einem resorbierbaren Material hergestellt werden kann.

In besonders vorteilhafter Weise kann dem erfindungsgemäßen Implantatmaterial ein pharmazeutischer Wirkstoff, wie ein Antibiotikum, und/oder ein Zytostatikum und/oder ein "Bone morphogenetic protein" und/oder ein Hämostyptikum und/oder ein Hormon, ein Chemotherapeutikum oder eine biologisch aktive Substanz zugesetzt werden. Hierzu ist Polyglykolat als Basismaterial besonders geeignet, da dessen Schmelztemperatur die Zerstörungstemperatur herkömmlicher Antibiotika, wie Gentamycin, nicht übersteigt. Implantatdübel mit derartigen Zusätzen führen beispielsweise bei Anwendung bei infizierten Pseudarthrosen nicht nur zur stabilen mechanischen Fixation, sondern auch zur wirksamen Lokaltherapie des Infektes.

Das erfindungsgemäße Implantat kann verschiedene äußere Gestaltungen aufweisen. Es ist wesentlich, daß das Implantat durch Aufspreizen, Dehnen oder Quellen in festen mechanischem Eingriff mit dem umgebenden Knochen kommt und den Knochen dadurch unterstützt und/oder eine Knochenschraube verankern kann. Zur Verbesserung des mechanischen Eingriffes ist die Außenfläche des Implantates vorzugsweise strukturiert, beispielsweise durch Schlitze, Vorsprünge und/oder Perforationen. Diese Strukturierung kann sich sowohl in Axialrichtung des Implantats, d.h. in der Richtung, in der das Implantat in den Knochen eingebracht wird, als auch senkrecht dazu in Umfangsrichtung des Implantats oder unter einem beliebigen Winkel erstrecken.

Die Strukturierung der Implantatoberfläche hat darüberhinaus den Vorteil, daß dadurch das Einwachsen des Knochens induziert wird. Allgemein kann man die Struktur von Implantaten in Strukturen erster bis vierter Ordnung einteilen. Nach dieser Definition entspricht die Struktur erster Ordnung dem äußeren Implantatdesign, die Struktur zweiter Ordnung stellt die Topographie des Implantats dar, beispielsweise besondere Oberflächengestaltungen, wie wellenartige oder sägezahnartige Gestaltungen der Implantatabschnitte, die Struktur dritter Ordnung umfaßt die Mikrostruktur der Oberfläche im Millimeter- oder Submillimeter-Bereich, und die Struktur vierter Ordnung stellt die Ultrastruktur der Oberfläche mit Strukturelementen in einer Größenordnung von etwa 10 - 200 µm, vorzugsweise 20 - 30 µm dar. Für optimale Knochenbildung und Knocheneinwuchs sind hierbei insbesondere die Strukturen dritter und vierter Ordnung maßgebend, und die erfindungsgemäßen Implantate weisen vorzugsweise eine Oberflächenstruktur dritter und vierter Ordnung auf.

Wenn das erfindungsgemäße Implantat als einseitig geschlossener, strukturierter Hohlzylinder ausgebildet ist, wird die Struktur dritter Ordnung vorzugsweise durch mehrere reifenartige Ringe oder Wülste um seinen Umfang ausgebildet. Vorzugsweise sind mehrere dieser Wülste in Axialrichtung des Zylinders regelmäßig und unmittelbar übereinander angeordnet, so daß sich etwa das Aussehen von übereinander geschichteten Reifen ergibt.

Der Durchmesser eines Ringquerschnittes oder der Ringabstand beträgt etwa 200 bis 3000 µm, vorzugsweise etwa 500 bis 2000 µm, die dem Knochen zugewandte Außenseite der Ringe ist etwa halbkreisförmig ausgebildet.

Alternativ zu dieser Ausgestaltung oder zusätzlich dazu kann auch eine Strukturierung in Form von Kugeln oder Kugelabschnitten, wie Halbkugeln mit einem Durchmesser von etwa 200 bis 2000 µm vorgesehen sein.

Zu einem beschleunigten Knocheneinwuchs kommt es insbesondere dann, wenn größere Kugeln mit kleineren Kugeln kombiniert werden. Ein Durchmesserverhältnis von 2:1 bis 3:1 ist besonders günstig. Beispielsweise können Kugeln mit einem Durchmesser von 200 bis 1000 µm, im Mittel 500 µm, mit Kugeln mit einem Durchmesser von 800 bis 3000 µm, im Mittel 1000 µm, vermischt sein. Vorzugsweise weisen die kleineren Kugeln einen Durchmesser von 300 bis 700 µm, besonders bevorzugt 450 - 550 µm, und die größeren Kugeln einen Durchmesser von 800 bis 2000 µm, besonders bevorzugt 900 - 1200 µm auf. Dabei weisen die kleineren Kugeln einerseits und die größeren Kugeln andererseits vorzugsweise alle etwa den gleichen Durchmesser, also z.B. 500 µm und 1000 µm auf. Um die kleineren Kugelelemente kommt es schneller zu einer Knochenanlagerung, während die tragenden Knochengewölbe sich um die großen Kugelelemente ausbilden.

Die Oberflächen der Kugeln oder Kugelabschnitte oder der Wülste können mikrostrukturiert sein, wobei die Mikrostrukturierung z.B. in Form von Kugeln oder Kugelabschnitten, wie Halbkugeln mit einem Durchmesser von 10 bis 50 µm, vorzugsweise 15 bis 30 µm ausgebildet ist.

Die Zwischenräume zwischen den größeren Kugeln oder Wülsten und/oder die Oberfläche des Implantats können mit einer Beschichtungsmasse ausgefüllt bzw. bedeckt sein, die zusammen mit einer etwaigen Mikrostrukturierung der größeren Kugeln oder Wülste die Struktur IV. Ordnung des erfindungsgemäßen Implantates darstellt. Die Beschichtungsmasse besteht dabei vorzugsweise aus einer organischen, meist resorbierbaren Matrix und anorganischen Füllerbestandteilen oder Füllkörpern.

Die Außenseite des Implantats kann entweder eine dicke Beschichtung mit einer Dicke von etwa 300 bis 2 000 µm oder eine dünne Beschichtung mit einer Dicke von etwa 20 bis 300 µm aufweisen. Die Schicht kann entweder geschlossen sein und das Implantat vollständig umfassen oder nur Teile des Implantates bedecken. Sie kann entweder direkt oder indirekt in einem Verbund aufgebracht sein.

Die Füllkörper bestehen vorzugsweise aus Kugelpartikeln mit einem Durchmesser von 10 bis 200 µm, vorzugsweise 15 bis 50 µm, besonders bevorzugt 15 bis 30 µm. Insbesondere die größeren Partikel sind zur Erleichterung des Einwachsens des Knochens vorzugsweise porös und weisen ein Porenvolumen von etwa 25 bis 65 % auf. Die genannten Kugeln werden von Osteoblasten als Basis erkannt und regen den Knocheneinwuchs an. Die Füllkörper bestehen vorzugsweise aus Tricalciumphosphat, Hydroxylapatit oder Bioglas.

Die Matrix der Beschichtung, die auch die Poren der hochporösen Füllkörper auffüllen kann, besteht vorzugsweise aus einem Polypeptid, Polylactat, Polyglycolat oder einem deren Cokondensate, Gelatine, Kollagen und/oder Calciumverbindungen.

Zumindest ein Teil der Füllkörper kann in Form von Fasern mit einer Dicke von 100 bis 300 µm, vorzugsweise etwa 200 µm und einer Länge von vorzugsweise mehr als 2 bis 15 mm ausgebildet sein, wobei die bevorzugte Länge mindestens 3 mm und höchstens 10 mm und die optimale Länge etwa 4 bis 5 mm ist. Die faserförmigen Füllkörper verbessern die Stabilität und bestehen vorzugsweise aus den gleichen Materialien wie die kugelförmigen Füllkörper. Die Fasern dienen, insbesondere nach dem Verstricken zu einem Gewebe, zur äußeren Armierung des Implantats.

In der Beschichtung kann ebenfalls zusätzlich ein pharmazeutischer Wirkstoff, beispielsweise ein Antibiotikum enthalten sein.

Besonders bevorzugt ist eine Mikrostrukturierung in Form von Kugeln in einem Verbund mit einer organischen Matrixschicht.

Die Erfindung wird nachstehend anhand der Zeichnung naher erläutert. Es zeigen:
- Figur 1: einen Längsschnitteiner Ausführungsform des erfindungsgemäßen Implantates in Form eines Knochendübels in schematischer Form,
- Figur 2: eine Seitenansicht eines erfindungsgemäßen Knochendübels, und
- Figuren 3 bis 9: weitere erfindungsgemäße Implantate.

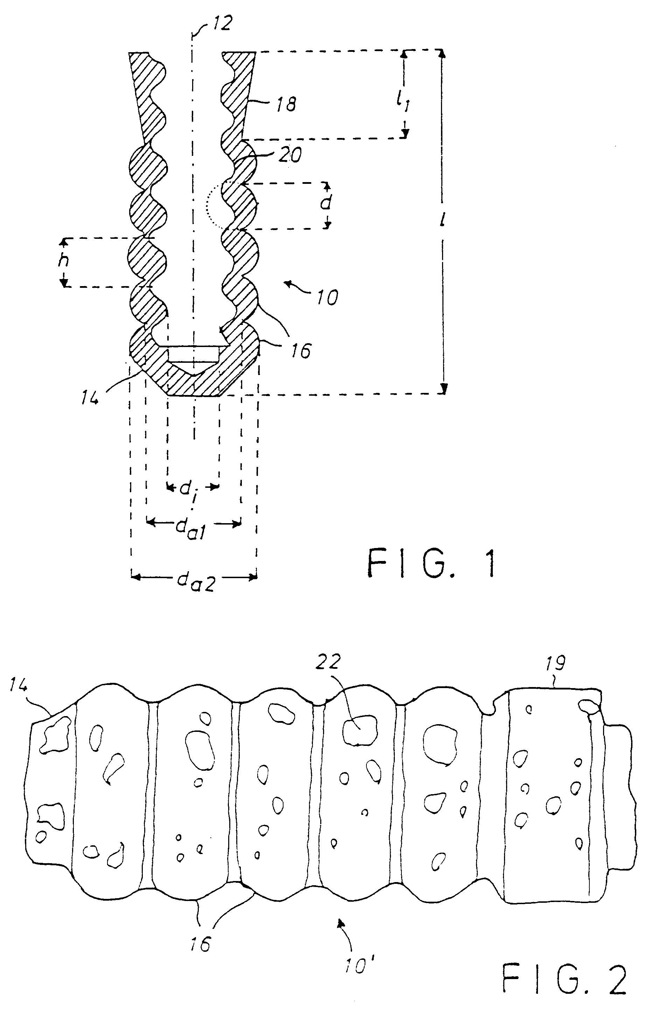

Das Implantat 10 gemäß Figur 1 stellt einen Knochendübel dar und weist die Form eines strukturierten Hohlzylinders mit einer Achse 12 auf. An seiner Spitze 14 ist der Knochendübel, um sein Einführen in den Knochen zu erleichtern, konusförmig ausgebildet, wobei der Konus mit der Achse 12 vorzugsweise einen Winkel von etwa 45° einschließt.

Entlang seines Umfangs weist der Knochendübel mehrere, vorzugsweise etwa fünf oder mehr Ringe oder Wülste 16 auf, die in Axialrichtung unmittelbar übereinander geschichtet sind. Der Dübel weist somit im Längsschnitt an seinem Umfang eine aus aneinandergereihten Halbkreisen bestehende Profilierung auf. Der hintere Endabschnitt 18 des Implantates 10 ist ebenfalls konusförmig ausgebildet, wobei der konusförmige Endabschnitt 18 mit der Achse 12 einen kleineren Winkel einschließt als die Spitze 14, beispielsweise etwa 10 bis 20°. Im Inneren des Implantates 10 ist ein Innengewinde 20 oder eine gewindeähnliche Struktur zum Einschrauben von Knochenschrauben, beispielsweise von Spongiosaschrauben ausgebildet.

Das Dübelimplantat 10 gemäß Figur 1 weist vorzugsweise etwa die folgenden Abmessungen auf, die an die Abmessungen üblicher Spongiosaschrauben angepaßt sind:
Länge l: 16 bis 32 mm
Außendurchmesser dₐ₂:6 bis 7 mm
Durchmesser dₐ₁: 4 bis 5 mm
Innendurchmesser dᵢ: 2,5 bis 3,5 mm
Wulstabstand d: 0,5 bis 3,0 mm
Ganghöhe h: 2,5 bis 3 mm
Länge l₁ des Endabschnittes: 4 bis 6 mm
Wandstärke: 0,5 bis 2,5 mm
Der Ringdurchmesser ist etwas größer als der Wulstabstand d (vgl. Punktierung in Fig. 1).

Die Abmessungen des Implantates hängen selbstverständlich vom Typ der zu verankernden Schraube oder von der Art des zu unterstützenden Knochens ab. Bei der Schraubenverankerung ist der Innendurchmesser des Implantates 10 vorzugsweise etwas kleiner als der Durchmesser der Knochenschraube, so daß das Implantat 10 beim Einschrauben der Schraube aufgespreizt wird. In zusammengedrücktem Zustand beim Einführen des Implantats beträgt der Außendurchmesser dₐ₂ etwa 4 bis 4,5 mm.

Das Implantat 10' gemäß Figur 2 entspricht weitgehend dem Implantat 10 gemäß Figur 1. Beim Implantat 10' ist der Endabschnitt 19 zylindrisch ausgebildet. Außerdem sind beim Implantat 10' Perforationen 22 auf seiner Außenfläche sichtbar. Zwischen den einzelnen Wülsten 16 kann das Implantat 10' kleine zylindrische Zwischenräume aufweisen.

Die Implantate 10 und 10' gemäß Fig. 1 und 2 sind aus Trikalziumphosphat-Polyglykolat ausgebildet, wobei der Trikalziumphosphat-Anteil beispielsweise etwa 25 % beträgt. Die Porosität der Oberfläche ist je nach Abkühlungsgeschwindigkeit abhängig von gasbildenden Zusätzen oder Gelen einstellbar. Sie kann aber auch durch die Gießform bis zur Struktur III. Ordnung reproduzierbar vorgegeben werden durch Zusatz von gasbildenden oder leicht verdampfbaren Substanzen.

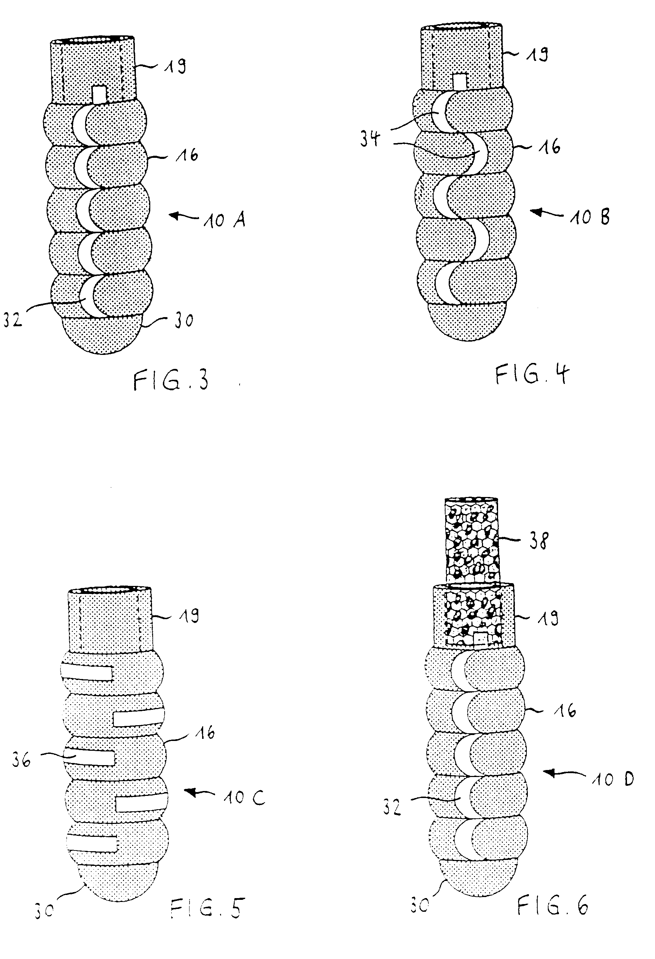

Das Implantat 10A gemäß Fig. 3 weist ebenfalls die Form eines strukturierten Hohlzylinders mit fünf Wülsten 16, einem zylindrischen Endabschnitt 19 und einem halbkugelförmigen vorderen Ende 30 auf. Ein in Längsrichtung des Implantats durchgehender Axialschlitz 32 erleichtert das Zusammendrücken des Implantats beim Einführen in den Knochen.

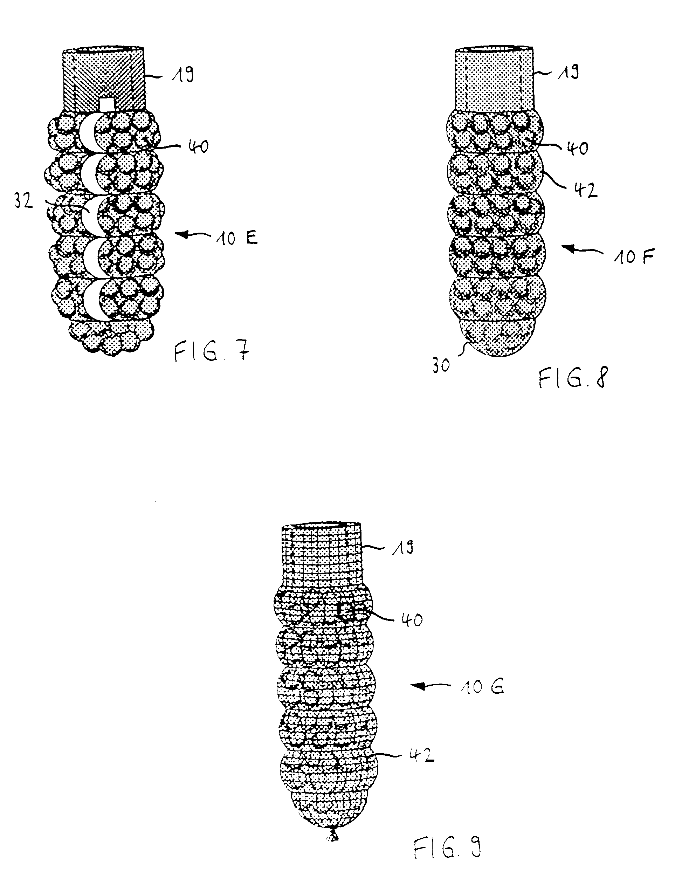

Das ebenfalls hohlzylinderförmige Implantat 10B gemäß Fig. 4 unterscheidet sich vom Implantat 10A dadurch, daß die Axialschlitze 34 der einzelnen Wülste 16 in Umfangsrichtung zueinander versetzt sind.

Beim Implantat 10C gemäß Fig. 5 weist jeder Wulst 16 einen Querschlitz 36 auf, der sich in Umfangsrichtung des Implantats erstreckt.

Um das Einführen des Implantats in den Knochen und sein Aufspreizen oder Aufquellen im Knochen weiter zu erleichtern, können die in den Fig. 3 bis 5 dargestellten Axial- und Querschlitze in geeigneter Weise miteinander kombiniert werden.

Das Implantat 10D gemäß Fig. 6 ist ebenfalls als hohlzylinderförmiger Dübel ausgebildet. In seinem inneren Hohlraum nimmt das Implantat einen autologen Knochenzylinder 38 auf, der mit Hilfe des Dübels stabil im Knochen fixiert werden kann. Das Implantat 10D ist resorbierbar ausgebildet, so daß ein rasches knöchernes Einheilen des reinserierten Knochenzylinders möglich ist. Statt des Knochenzylinders 38 kann das Implantat 10D auch beispielsweise einen Medikamenten- und/oder Strahlentherapieträger aufnehmen.

Das Implantat 10E gemäß Fig. 7, das ebenfalls einen Längsschlitz 32 aufweist, ist aus einem Matrix-Kugelverbund aus sich berührenden Kugeln 40 aufgebaut. Der Kugelverbund wird durch die Beschichtungsmasse zusammengehalten.

Beim Implantat 10F gemäß Fig. 8 wird die in einem Verbund stehende Kugelpackung aus den Kugeln 40 von einem Netz 42 armiert. Das Netz 42 kann beispielsweise ebenfalls aus verstrickten Kugelketten oder aus zu einem Netz verbundenen extrudierten Fasern oder Fäden ausgebildet sein, die gegebenenfalls mit kleinen Kugeln bestückt sind.

Beim Implantat 10G gemäß Fig. 9 sind zunächst lose Kugeln 40 in einem aus verstrickten Fasern oder Fäden ausgebildeten, doppelwandigen Strumpfkäfig 42 gefangen. Durch Verdrehen des Strumpfkäfigs 42 können die Kugeln in engen gegenseitigen Kontakt gebracht werden. Nach dem Einbringen in den Knochen quillt das Implantat auf und kann durch Eindrehen einer Schraube oder Einführen eines Zylinders oder Keiles in seinen inneren Hohlraum aufgespreizt und im Knochen verblockt werden.

Die mit Kunststoffdübeln verankerten Knochenschrauben zeigen im Versuch an Schweineepiphysen, bei denen Osteotomien gesetzt worden waren, eine um mehr als 50 % erhöhte Zugfestigkeit. Dabei ist zu bemerken, daß die Spongiosa der Schweineepiphysen eine sehr kompakte und feste Knochenstruktur darstellt. Da die Zugfestigkeit der ohne Dübel fixierten Spongiosaschrauben am menschlichen Knochen, vor allem am älteren Knochen, nahezu Null ist, ergibt sich bei der Verwendung der erfindungsgemäßen Dübel-Implantate zur Verankerung von Spongiosaschrauben im menschlichen Knochen eine beträchtliche Erhöhung der Zugfestigkeit, gegebenenfalls um ein Vielfaches. Im Tierversuch wird die Zugfestigkeit der Spongiosaschrauben in Abhängigkeit von der Zeit dadurch gemessen, daß den Schrauben Kraftmesser vorgeschaltet werden.

Mit resorbierbaren Dübeln ergeben sich von der Zugfestigkeit her gesehen ähnliche Ergebnisse wie mit Kunststoffdübeln.

Die Beispiele erläutern die Erfindung anhand der Herstellung von Ausführungsformen der erfindungsgemäßen Implantate.

### Beispiel 1

In einem Schmelztiegel werden 2g Polyglykolat-Granulat mit einigen Gramm bioverträglichem Weichmacher versetzt und bei ca. 130^{o}C geschmolzen. In die heiße Schmelze gibt man 0,5g fein pulverisiertes Tricalciumphosphat und rührt solange, bis eine homogene Masse entsteht. Die heiße Schmelze wird anschließend in eine Metallform gegossen, die feinmechanisch aus Aluminium oder Messing gefräst wurde. Eine Dübelform mit einem Hohlzylinder mit Innengewinde wird in einfacher Weise dadurch hergestellt, daß man eine handelsübliche Knochenschraube, deren Gewinde um 1-2mm abgefräst wurde, in die mit heißer Schmelze gefüllte Metall-Gußform einpaßt. Nach Erkalten der Schmelze wird der Dübel mit der eingelegten Schraube von der Metallgußform befreit. Die Knochenschraube wird anschließend aus der erkalteten Schmelze herausgedreht.

### Beispiel 2

Zur Herstellung eines Knochendübels mittels Spritzgußverfahren wird aus einem Polyglykolat-Granulat und einigen Gramm bioverträglichem Weichmacher in einer eigens dafür vorgesehenen Apparatur eine Schmelze hergestellt. Nach Erreichen einer homogenen Masse wird die flüssige Schmelze mittels Drucken um 20 bar in eine Spritzgußform eingespritzt und anschließend erkalten lassen. Die Porösität der Oberfläche kann durch die Abkühlgeschwindigkeit reguliert werden. Großporige Oberflächenstrukturen können durch Zusatz von Gelen oder leichter flüchtigen Reagentien erzeugt werden (Struktur IV. Ordnung), während die Strukturen II. und III. Ordnung am einfachsten durch Gestaltung der Gußform gelöst werden können.

## Patentansprüche

1. Implantat zur Armierung von Knochen und zur Verankerung von Knochenschrauben, Implantaten oder Implantatteilen im Knochen, welches aus einem resorbierbaren Material besteht und derart als Hohlkörper ausgebildet ist, daß es in den Knochen einbringbar ist, und welches eine solche Form aufweist und/oder aus einem solchen Material gebildet ist, daß es im Knochen aufspreizbar und/oder aufquellbar ist, und dadurch einen festen Halt im umgebenden Knochen findet.

2. Implantat zur Armierung von Knochen und zur Verankerung von Knochenschrauben, Implantaten oder Implantatteilen im Knochen, welches aus einem resorbierbaren Material besteht und in den Knochen einbringbar ist, und welches eine solche Form aufweist und/oder aus einem solchen Material gebildet ist, daß es in den Knochen derart einschraubbar ist, daß es einen festen Halt im umgebenden Knochen findet.

3. Implantat nach Anspruch 1 oder 2, dadurch gekennzeichnet, daß seine Oberfläche strukturiert ist, z.B. durch Schlitze (32,34,36) und/oder Perforationen (22) und/oder Erhebungen, Gewindegänge und/oder gewindeähnliche Erhebungen, Poren und/oder Mikrorauhigkeiten.

4. Implantat nach einem der Ansprüche 1 bis 3, dadurch gekennzeichnet, daß es im wesentlichen als dübelartiger Hohlzylinder (10) ausgebildet ist, der an seiner Außenseite mehrere, in Axialrichtung des Hohlzylinders (10) übereinander angeordnete ringartige Wülste (16) und/oder sich berührende Kugeln (40) oder Halbkugeln trägt, wobei das Profil der Wülste (16) vorzugsweise etwa halbkreisförmig und/oder halbkugelförmig ist, und der Durchmesser der Wülste (16) oder der Kugeln oder Halbkugeln vorzugsweise etwa 200 bis 3 000 µm beträgt.

5. Implantat nach einem der Ansprüche 1 bis 4, dadurch gekennzeichnet, daß es eine Spitze oder ein halbkugelförmiges Ende (30) oder konisches Ende (14) und/oder daß es ein Innengewinde (20) und/oder eine Innenstruktur und/oder eine gewindeähnliche Innenstruktur aufweist.

6. Implantat nach einem der Ansprüche 1 bis 5, dadurch gekennzeichnet, daß seine äußere Oberfläche mikrostrukturiert ist, wobei die Mikrostrukturierung vorzugsweise Kugeln oder Halbkugelflächen mit einen Durchmesser von 10 - 50 µm, vorzugsweise 20 - 30 µm aufweist, und/oder daß die Mikrostrukturierung der Kugeln in einem Verbund mit einer Matrixschicht besteht und daß als Matrix vorzugsweise ein organisches Material auf der Basis eines Kollagens, Polyglykolates, Polylactates oder eines Polypeptids verwandt wird.

7. Implantat nach einem der Ansprüche 1 bis 6, dadurch gekennzeichnet, daß es aus anorganischen oder organischen Materialen und/oder aus Verbunden aus resorbierbaren anorganischen und/oder organischen Materialien besteht, wobei das resorbierbare organische Material vorzugsweise ein Stoff auf der Basis von Polylactaten, Polyglykolaten oder Polypeptiden oder Kollagenen oder aus Verbunden verschiedener organischer Materialien und/oder Verbunden aus organischen Materialien mit anorganischen resorbierbaren Materialien, wie Apatiten oder resorbierbaren Keramiken ist und das resorbierbare anorganische Material ein Apatit, ein Trikalziumphosphat oder eine resorbierbare Keramik ist.

8. Implantat nach Anspruch 7, dadurch gekennzeichnet, daß es aus einem Verbund aus einer resorbierbaren Matrix und einem resorbierbaren Füller besteht, der vorzugsweise in Form eines resorbierbaren Trikalziumphosphates oder eines Hydroxylapatites beigesetzt ist, und vorzugsweise in Form von Kugeln mit einer Größe von etwa 10 - 200 µm, vorzugsweise 20 - 30 µm ausgebildet ist, und/oder daß die Oberfläche der Matrix des Implantats durch eingelagertes Tricalciumphosphat oder Hydroxylapatitpulver aufgerauht ist.

9. Implantat nach Ansprüche 7 oder 8, dadurch gekennzeichnet, daß das Material durch eine Faser-, Fasernetz- oder Gewebearmierung (42) aus einem resorbierbaren Faden oder Nahtmaterial verstärkt ist.

10. Implantat nach einem der Ansprüche 1 bis 9, dadurch gekennzeichnet, daß zusätzlich ein pharmazeutischer Wirkstoff enthalten ist, z.B. ein Antibiotikum, wie Gentamycin, und/oder ein Zytostatikum und/oder ein "Bone morphogenetic protein" und/oder ein Hämostyptikum und/oder ein Hormon.

11. Implantat nach den Ansprüchen 1 bis 10, dadurch gekennzeichnet, daß es an der Außenseite in dicken und/oder dünnen Schichten beschichtet ist und daß die Schichten vorzugsweise aus einer organischen Matrix mit anorganischen Füllerbestandteilen bestehen, und/oder daß die Schicht aus einem Trikalziumphosphat, Bioglas oder Hydroxylapatit in geschlossener oder nicht geschlossener Form besteht und diese Schicht direkt oder indirekt in einem Verbund aufgebracht ist.

12. Implantat nach Ansprüch 11, dadurch gekennzeichnet, daß die Matrix der organischen Beschichtung aus einem Kollagen, Polyglykolat, Polylactat oder einem Polypeptid besteht, und/oder daß in der Beschichtung ein pharmazeutischer Wirkstoff enthalten ist, z.B. ein Antibiotikum, und/oder Zytostatikum und/oder "Bone morphogenetic protein" und/oder Hämostyptikum.

13. Implantat nach den Ansprüchen 1 bis 12, dadurch gekennzeichnet, daß Kugeln der Größe 500 - 5 000 µm, vorzugsweise 1 000 - 3 000 µm, in einem doppelwandigen Strumpfkäfig gefangen sind, so daß ein Lumen ausgebildet ist, in das eine Schraube eindrehbar oder ein Zylinder oder Keil verblockbar ist.

## Claims

1. Implant to sheath bone and to anchor bone screws, implants or implant parts in bone, said implant consisting of an absorbable material and being provided as a hollow body such that it is insertable into bone and being in such a shape and/or of such a material that it can be expanded and/or swelled in bone and is thus kept firmly in place in surrounding bone.

2. Implant to sheath bone and to anchor bone screws, implants or implant parts in bone, said implant consisting of an absorbable material and being insertable in bone and being in such a shape and/or of such a material that it can be screwed into the bone such that it is kept firmly in place in surrounding bone.

3. Implant according to claim 1 or 2, characterized in that its surface is structured, e.g. by grooves (32, 34, 36) and/or perforations (22) and/or projections, threads and/or thread-like projections, pores and/or microcoarsenesses.

4. Implant according to any one of claims 1 to 3, characterized in that the shape of the implant is essentially that of a dowel-like hollow body (10) which carries several stacked ring-like bulges (16) and/or continguous spheres (40) or semispheres along the axis of the body's outer surface, wherein the profile of the bulges (16) is preferably approximately semicircular and/or hemispherical, and the diameter of the bulges (16) or the spheres or semispheres is preferably between approximately 200 and 3000 µm.

5. Implant according to any one of claims 1 to 4, characterized in that the implant comprises a tip or a hemispherical end (30) or conical end (14) and/or that it comprises an internal thread (20) and/or an internal structure and/or a thread-like internal structure.

6. Implant according to any one of claims 1 to 5, characterized in that the outer surface of the implant is microstructured, wherein the microstructure preferably comprises spheres or hemispherical surfaces with a diameter of 10-50 µm, preferably 20-30 µm, and/or that the microstructure consists of spheres in a composite with a matrix layer and that an organic material with a collagen, polyglycolate, polylactate or polypeptide base is preferably used as a matrix.

7. Implant according to any one of claims 1 to 6, characterized in that the implant consists of inorganic or organic materials and/or of composites of absorbable inorganic and/or organic materials, wherein the absorbable organic material is preferably a substance with a polylactate, polyglycolate or polypeptide or collagen base or consists of composites of various organic materials and/or composites of organic materials with inorganic absorbable materials such as apatites or absorbable ceramics and the absorbable inorganic material is an apatite, a tricalcium phosphate or an absorbable ceramic.

8. Implant according to claim 7, characterized in that the implant consists of a composite of an absorbable matrix and an absorbable filler, the latter preferably being added in the form of an absorbable tricalcium phosphate or a hydroxylapatite, and preferably being in the form of spheres approximately 10 - 200 µm in size, preferably 20 - 30 µm in size, and/or that the surface of the matrix of the implant is roughened by embedded tricalcium phosphate or hydroxylapatite powder.

9. Implant according to claim 7 or 8, characterized in that the material is reinforced by a fiber, fibrous network or tissue sheath (42) consisting of an absorbable thread or suture material.

10. Implant according to any one of claims 1 to 9, characterized in that an active pharmaceutical agent is also contained, e.g. an antibiotic, such as gentamycin, and/or a cytostatic agent and/or a bone morphogenetic protein and/or a hemostatic agent and/or a hormone.

11. Implant according to any one of claims 1 to 10, characterized in that the outer side of the implant is coated with thick and/or thin layers and that the coats preferably consist of an organic matrix with inorganic filler components, and/or that the layer consisting of a tricalcium phosphate, bioglass or hydroxylapatite is closed or non-closed and that this layer is applied directly or indirectly in a composite.

12. Implant according to claim 11, characterized in that the matrix of the organic coating consists of a collagen, polyglycolate, polylactate or a polypeptide, and/or that in the coating a pharmaceutical substance is contained, e.g. an antibiotic, such as gentamycin, and/or a cytostatic agent and/or bone morphogenetic protein and/or a hemostatic agent.

13. Implant according to claims 1 to 12, characterized in that spheres of 500-5000 µm, preferably 1000-3000 µm in size are held in a double-walled stocking cage, thus forming a lumen into which a screw can be screwed or a cylinder or wedge can be locked.

## Revendications

1. Implant de renforcement d'os et d'ancrage de vis osseuses, d'implants ou de parties d'implant dans les os, qui est constitué d'un matériau capable de absorption et se présente sous la forme d'un corps creux de telle sorte qu'il puisse être inséré dans l'os, et qui présente une forme telle et/ou est formé d'un matériau tel qu'il puisse s'expanser et/ou se gonfler dans l'os, et soit ainsi fermement maintenu dans l'os qui l'entoure.

2. Implant de renforcement d'os et d'ancrage de vis osseuses, d'implants ou de parties d'implant dans les os, qui est constitué d'un matériau capable de absorption et peut être inséré dans l'os, et qui présente une forme telle et/ou est constitué d'un matériau tel qu'il puisse être vissé dans l'os et soit ainsi fermement maintenu dans l'os qui l'entoure.

3. Implant selon la revendication 1 ou 2, caractérisé en ce que sa surface est structurée, par exemple par des fentes (32, 34, 36) et/ou des perforations (22) et/ou des reliefs, des rainures spiralées et/ou des reliefs spiralés, des pores et/ou des micro-rugosités.

4. Implant selon l'une des revendications 1 à 3, caractérisé en ce qu'il est en substance réalisé sous la forme d'un cylindre creux (10) formant une cheville, qui porte sur sa face extérieure plusieurs bourrelets (16) annulaires disposés les uns derrière les autres dans la direction axiale du cylindre creux (10) et/ou des billes (40) ou des demi-billes se touchant, le profil des bourrelets (16) étant de préférence en forme approchée de demi-cercles et/ou hémisphérique, et le diamètre des bourrelets (16) ou des billes ou des demi-billes valant de préférence entre environ 200 et 3000 µm.

5. Implant selon l'une des revendications 1 à 4, caractérisé en ce qu'il présente une pointe ou une extrémité (30) de forme hémisphérique ou une extrémité (14) conique et/ou en ce qu'il présente un filet intérieur (20) et/ou une structure intérieure et/ou une structure intérieure de forme spiralée.

6. Implant selon l'une des revendications 1 à 5, caractérisé en ce que sa surface extérieure est microstructurée, la microstructuration présentant de préférence des billes ou des surfaces hémisphériques d'un diamètre de 10-50 µm, et de préférence de 20-30 µm, et/ou en ce que la microstructuration est constituée de billes dans un composite avec une couche de matrice et en ce que l'on utilise pour la matrice de préférence un matériau organique à base d'un collagène, d'un polyglycolate, d'un polylactate ou d'un polypeptide.

7. Implant selon l'une des revendications 1 à 6, caractérisé en ce qu'il est constitué d'un matériau inorganique ou organique et/ou d'un composite de matériau inorganique et/ou organique capable d'absorption, le matériau organique capable d'absorption étant de préférence un tissu à base de polylactate, de polyglycolate ou de polypeptide ou de collagène ou de composite de différents matériaux organiques et/ou de composites de matériaux organiques avec matériaux inorganiques capables d'absorption, comme l'apatite ou des céramiques capables d'absorption, et le matériau inorganique capable d'absorption étant une apatite, un phosphate tricalcique ou une céramique capable d'absorption.

8. Implant selon la revendication 7, caractérisé en ce qu'il est constitué d'un composite d'une matrice capable d'absorption et d'un agent de remplissage capable d'absorption, qui est utilisé de préférence sous forme d'un phosphate tricalcique capable d'absorption ou d'une hydroxylapatite, et de préférence sous forme de sphères d'une taille d'environ 10-200 µm, et de préférence de 20-30 µm, et/ou en ce que la surface de la matrice de l'implant est rendue rugueuse par un phosphate tricalcique ou une poudre d'hydroxylapatite à phase dispersée.

9. Implant selon la revendication 7 ou 8, caractérisé en ce que le matériau est renforcé par une armature de fibres, d'un réseau de fibres ou d'un tissu (42) constitué de fils ou d'un matériau de ligature capables d'absorption.

10. Implant selon l'une de revendications 1 à 9, caractérisé en ce qu'en plus il contient un agent pharmaceutique, par exemple un antibiotique tel que par exemple la gentamycine et/ou un agent cytostatique et/ou une "protéine morphogénétique de l'os" et/ou un agent hémostyptique et/ou une hormone.

11. Implant selon les revendications 1 à 10, caractérisé en ce que sa face extérieure est enduite de couches épaisses et/ou fines et en ce que les couches sont de préférence constituées d'une matrice organique avec composant inorganique de remplissage, et/ou en ce que la couche est constituée d'un phosphate tricalcique, d'un bioverre ou d'une hydroxylapatite sous forme fermée ou non fermée, et en ce que cette couche est apportée directement ou indirectement dans un composite.

12. Implant selon la revendication 11 , caractérisé en ce que la matrice de l'enduit organique est constituée d'un collagène, d'un polyglycolate, d'un polylactate ou d'un polypeptide, et/ou en ce qu'un agent pharmaceutique est contenu dans l'enduit, par exemple un antibiotique tel que par exemple la gentamycine, et/ou un agent cytostatique et/ou une "protéine morphogénétique de l'os" et/ou un hémostyptique.

13. Implant selon les revendications 1 à 12, caractérisé en ce que les billes d'une taille de 500-5000 µm, de préférence 1000-3000 µm sont reprises dans une enceinte en forme de manchon à double paroi de telle sorte que soit formée une ouverture dans laquelle une vis peut être insérée par rotation ou un cylindre ou un piton conique peut être enfoncé et serré.
